# EUROPEAN PATENT APPLICATION

(11) **EP 4 473 942 A1**
(43) Date of publication of application: **11.12.2024**
(21) Application number: 22924682.2
(22) Date of filing: 27.12.2022
(51) Int. Cl.: A61F 2/01

(54) **EMBOLIC PROTECTION DEVICE HAVING A DOUBLE CONICAL SPIRAL FOR EMBOLIC PROTECTION DURING ENDOVASCULAR PROCEDURES**

(30) Priority: 03.02.2022 ES 202230080
(71) Applicant: Fundación para la Investigación del Hospital Universitario y Politécnico la Fe de la comunidad Valenciana, 46026 Valencia (ES)
(72) Inventor: MIRALLES HERNÁNDEZ, Manuel, 46025 Valencia (ES)
(74) Representative: Clarke, Modet y Cía., S.L.
(86) International application number: PCT/ES2022/070836
(87) International publication number: WO 2023/148413

(57) **Abstract**

Embolic protection device (1) inside a blood vessel (10) comprising a filament preformed as a double conical spiral structure with a first peripheral zone (2) formed by a floppy (3) followed by a first set of coils (4) in the form of a cone; a central zone (5) formed by at least one central coil and a second peripheral zone (6), following the central zone (5), formed by a second set of coils (7) and a terminal means (8) located at the end of the second set of coils (7), wherein the second set of coils (7) is movable from an open position to a closed position, forming a cone of ascending diameter in the direction of blood flow inthe openposition , and a cone of descending diameter in the direction of blood flow through the central zone (5) in the closed position.

## Description

### Field of the Invention

The present invention is in the healthcare sector, specifically in those devices used for temporary embolic protection during the practice of endovascular procedures.

### Prior art

Angioplasty and stenting are now common procedures to restore adequate blood flow in blood vessels. However, embolization of detached fragments of atheroma plaque is a frequent phenomenon during angioplasty procedures, carotid or in other vascular territories. Particles larger than 100 microns are of special interest in this regard, due to the significant lesions they can produce in the brain. Currently, there are different Embolic Protection Devices (EPD), which can be broadly classified as solutions based on:
- distal occlusion and retrograde aspiration,
- spontaneous or forced flow reversal, and
- network type.

The most commonly used are network type solutions. These EPDs consist of a basket-like mesh or filter mounted on a nitinol ring that is advanced by a low-profile catheter and deployed distal to the lesion to be treated. Subsequently, they are retrieved by means of a catheter with a higher profile and suitable for each model. The advantages of these network-type EPDs over the other types include active protection by mechanical interception capture of the particles maintaining uninterrupted antegrade cerebral flow during the procedure. Disadvantages include difficulty or risk of fragmentation when passing through the lesion, possibility of damage to the vessel wall by the carrier ring and particle leakage due to low efficiency after deployment or during filter removal.

Among the currently existing solutions that present similarities with the object of the invention, the one provided in document US2007100373A1 can be highlighted. It refers to a filter for the retention of clots that presents a nitinol structure, covered with a filtering mesh, which may be made of nylon. Among its embodiments, a solution is described wherein the nitinol structure presents a conical spiral shape.

WO2021087480A1 refers to an embolic protection device that includes a filtering system to prevent clots from reaching the brain. Filter porosity can be 40, 100, 150, 200 or 300 microns, or any porosity in between. The expandable frame of the filter element may be made of nitinol (0,010 m diameter), and the filter material may be a perforated polymer film, a woven or knitted mesh material, or other material with a specific porosity.

US2019125513A1 refers to a filter (33) with a frame and a filter element having pores configured to allow blood to flow through it, but which are small enough to prevent embolic particles from passing through it. The pores can be between 10 microns and 1 mm in diameter. Among the embodiments described in the paper, the frame may be composed of a material such as nitinol and present a spiral shape.

US2018289460A1 discloses an embolic protection filter with a 6 French (2mm thick) guide catheter. Among one embodiment, a solution composed of two filters is described, where the more distal filter has a larger diameter and with a pore size of 50-200 microns, will collect the smaller clots. The structure of the filters is made of nitinol and would be coated with materials such as nylon.

These devices have different conical designs of filtering mesh for placement in medium or large caliber vessels, which translates into the need for carrier catheters of larger diameter, generally greater than 3 F. In addition, current solutions present problems to be solved, such as resistance to flow exerted by the device, variable particle retention efficiency, especially due to poor adaptation to the vessel of the circular frame that supports the mesh, and leaks during folding and removal of the mesh

On the other hand, there are currently other solutions that do not require a filtering mesh, and it is the structure itself that acts as a particle filter. Among these solutions, we find document WO2008127328A1, where a device for clot capture is described, where numerous embodiments are described, among which we can highlight a embodiment with a cylindrical shape in its central part and ending in a distal conical section, manufactured in nitinol.

Despite the multiple solutions of embolic protection devices currently available, they are not able to solve all the challenges that exist in this type of process.

On the other hand, there are other solutions such as the device described in documents US2008004640A1, where a device for the closure of a blood vessel access with a double helix structure is described. Although it presents a solution-like structure, such a structure is not capable of providing a device with a filter function effectively during the practice of endovascular procedures.

Therefore, among the drawbacks of current solutions for an embolic protection device can be highlighted:
- Particulate embolic capture efficiency limited by filter structure and geometry
- Flow resistance exerted by the device,
- Leakage of particles during pleating and filter removal

There is, therefore, a need for a solution to improve embolic protection devices.

### Description of the Invention

In order to solve the drawbacks existing in the current state of the art, a new embolic protection device is presented.

The present invention consists of a device for the capture of embolic material (clots, plaque fragments, etc.), generated during the practice of endovascular procedures. This device comprises a preformed filament with a double conical spiral structure.

In the present memory, a preformed filament is understood as a filament that has the ability to remember an initial shape and is capable of returning to the same shape once it has been deformed. In general, shape recovery can be caused by a thermal or magnetic change. There are many materials with this capability, including nitinol, an alloy of nickel and titanium in different proportions, which has excellent shape memory properties, as well as good electrical and mechanical properties, fatigue and corrosion resistance.

For this reason, in a preferred embodiment of the invention, the filament is manufactured from nitinol. However, in an obvious manner, the filament may be made of another material equivalent to nitinol, which makes the filament suitable for use in a device for embolic material capture inside a blood vessel.

Therefore, in the initial state, the filament has a rectilinear shape, to facilitate its entry into the target vessel through a carrier catheter. Once the filament is deployed, i.e., after passing the tip of the carrier catheter used for entry into the vessel, due to the increase in temperature caused by contact with the blood, the filament recovers the preformed double conical spiral arrangement.

Thus, in use, the preformed filament has a double conical spiral structure. Said structure comprises a first peripheral zone, a central zone and a second peripheral zone.

The first peripheral zone is the beginning of the filament as it enters the vessel. It is formed by a first straight end, called floppy, of soft consistency; followed by a first set of cone-shaped coils of descending diameter in the direction of blood flow.

The central zone is formed by at least one loop and is configured to contact the walls of the blood vessel. The at least one spiral in the central zone has the maximum diameter of the device, serving as a base for the first and second peripheral zones.

The second peripheral zone is formed by a second set of coils, downstream of the at least one coil of the central zone, and a terminal means, configured to control the deployment and withdrawal of the device. The second set of coils presents, again, a cone shape with the base of the cone being the central zone coil and the diameter of the coils descending to the middle terminal.

Additionally, the preformed filament structure of the device is not fixed, but the second peripheral zone is movable from an open position, where the set of coils forms a cone whose base is the central zone and the second set of coils move away from this zone, to a closed position, where the cone formed by the second set of coils is introduced inside the central zone and/or the first peripheral zone.

Thus, in the initial position, or open position, the second set of coils presents an ascending diameter of its coils in the direction of blood flow, forming a cone protruding from the central area. In other words, in the open position, the filament has a central area, of maximum diameter, which serves as a common base for the two opposing peripheral cones. However, in the closed position, the cone formed by the second set of coils moves to the interior of the central zone and/or first peripheral zone, reversing the direction of the cone, and the set of coils then has a descending diameter in the direction of blood flow.

This displacement can be generated by the action of the blood flow itself, which pushes the second set of coils, or, alternatively, the terminal means can be employed to facilitate the displacement of the second peripheral zone from the open to the closed position in a controlled manner.

Therefore, in use, the filament presents a double conical spiral structure, where the central zone contacts the wall and the closed position of the second peripheral zone allows to obtain a structure where both cones are oriented in favor of the blood flow of the vessel. In this situation, the reduced distance between the set of coils of the first peripheral zone and the second peripheral zone allows the current device as a filter of the blood flow through it, with the maximum possible adaptability to the vascular lumen, even in curved areas, thanks to the central zone of the device.

The nitinol filament (or equivalent material) has a diameter between 300 - 500 microns. The use of a small filament diameter favors the application of the device by requiring a smaller diameter of the catheter used to introduce the device into the blood vessel. On the other hand, this filament has a semi-rigid consistency. In other words, it is a malleable filament, which recovers its rigid form inside the blood vessel, with sufficient consistency to support the blood flow that passes through it. The distance between each of the first and second set of coils are predefined to be less than or equal to the target particles. In this sense, according to this configuration, since the main objective of the device is the capture and retention of particles of less than 100 microns, the separation distance between the coils, once the preformed structure is formed, is less than or equal to 100 microns.

In a second aspect of the invention, the device may comprise a fibrillated filament. That is, the preformed filament further has a set of fibers, preferably nylon fibers, along the length of the filament. These fibers, with a length of 100 - 200 microns, are preferably anchored every 50 - 100 microns perpendicularly to the nitinol filament. In this way, the space between the coils is limited.

By incorporating the fibers into the filament, the capture capacity of the initial filament is increased. In this sense, it is possible to increase the required distance between the turns of the filament, thus reducing the amount of nitinol used (or equivalent material) to obtain this type of embolic protection devices for an equal diameter of the central area. Thus, the distance between the coils can be increased up to 200 microns for devices aiming to capture particles smaller than 100 microns.

In contrast to the prior art, where the use of designs with conical spiral either at one or both ends, they do not describe the formation of a filter with a double overlapping spiral, capable of being employed as a filter for embolic protection. This configuration also allows the use of smaller filaments than those used in the technique, resulting in the use of smaller catheters, such as low-profile (micro)catheters (less than 2F), which are less invasive during this type of procedure.

On the other hand, the use of a fibrillated filament provides an alternative to the use of current devices, such as devices with nylon mesh, which has a better capture of solid particles in the blood stream and prevents leakage during device removal. Additionally, the present solution presents a lower resistance to blood flow, coupled with a more consistent retention efficiency. The increase in retention efficiency is achieved mainly by the improved adaptation to the vessel of the circular frame that supports the mesh, and the reduction of leakage during folding and removal of the mesh.

Therefore, the present invention achieves:
- High capacity to capture particles, even of small sizes
- Maximum adaptability to the vascular lumen, even in curved areas, preventing particle leakage during movement and removal of the device
- Minimal resistance to blood flow
- Reduced advancement and withdrawal profile, requiring a smaller caliber of the carrier catheter due to the reduced thickness of the filament

In the figures, the following elements are shown:
1. Embolic protection device
2. First peripheral zone of the filament
3. Floppy
4. First set of loops
5. Central zone
6. Second peripheral zone
7. Second set of coils
8. Terminal means
9. Filament fibers
10. Blood vessel

Throughout the description and claims the word "comprises" and variants thereof are not intended to exclude other technical features, components or steps. In addition, the word "comprises" includes the case "consists of". To those skilled in the art, other objects, advantages and features of the invention will be apparent in part from the description and in part from the practice of the invention. The following examples and drawings are provided by way of illustration, and are not intended to be limiting of the present invention. Furthermore, the present invention covers all possible combinations of particular and preferred embodiments indicated herein.

### Brief description of the drawings

Figure 1 shows a perspective view of a first preferred embodiment of the fiberless embolic protection device.
Figure 2 shows a perspective of a second preferred embodiment of the fibrillated embolic protection device in the open position.
Figure 3 shows a perspective of a second preferred embodiment of the fibrillated embolic protection device in the closed position.
Figure 4 shows a section of the open position of the embolic protection device.
Figure 5 shows a section of the closed position of the embolic protection device.

### Detailed description of the invention

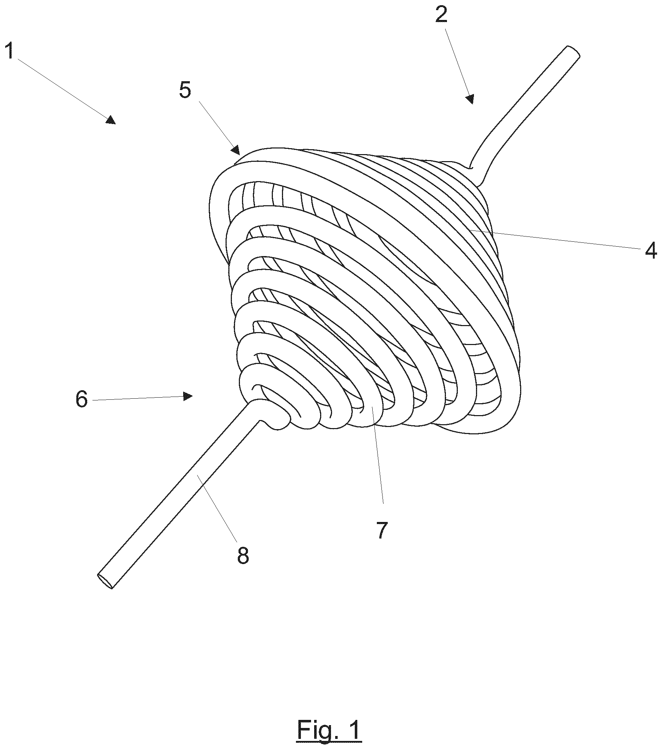

Figure 1 shows a perspective of a first preferred embodiment of the embolic protection device.

As can be seen in this figure, in this first preferred embodiment, the embolic protection device (1) comprises a filament preformed as a double conical spiral structure for clot capture inside a blood vessel (10). Additionally, the filament is semi-rigid, that is, it is a filament made of a malleable material, which recovers a rigid shape inside the blood vessel (10), being able to maintain its shape in the presence of blood flow (usually less than 300 ml/min).

The structure shown in Figure 1 is a preformed configuration of an initially rectilinear filament. In use, the rectilinear filament is introduced inside a blood vessel (10) guided by a floppy (3) located at its proximal end, with the aid of a catheter. The entry of the filament into a blood vessel (10) provides sufficient thermal input for the filament to regain the preformed arrangement with a double conical spiral structure. As an alternative to this embodiment, where the filament is made of nitinol, solutions can be presented with a material such that a preformed shape can be recovered inside a blood vessel.

In this double conical spiral structure, a first peripheral zone (2) can be distinguished. This zone is formed by a first set of coils (4) in the form of a cone, which presents, at its end, the floppy (3). In this way, the set of coils (4) forms a cone, where the coils reduce their diameter in the direction of the blood flow of the blood vessel (10), up to their junction with the floppy (3).

Preferably, in order to favor the entry of the device (1) inside the blood vessel (10) the diameter of the filament is between 300 - 500 microns. In addition, the preformed structure presents a distance between each of the coils of the first set of coils (4) of less than 100 microns, achieving the capture and retention of particles of this type of size. although the distance between coils is less than 100 microns, the conical arrangement of these coils manages to reduce the passage space of the first set of coils (4) in the direction of blood flow to 40 microns.

Thus, the embolic protection device can be used as a filter to capture clots inside a blood vessel (10).

As discussed above, the filament of the device (1) has a floppy (3) at its end. This element, with a length of 2 - 3 cm preferably, allows aligning the rest of the structure with the blood vessel (10) where it is introduced, thus avoiding the injuries that would occur in this type of applications.

For its part, the base of the cone is joined to the central area (5) of the structure, which is formed by at least one central spiral. In this particular embodiment, the central zone (5) consists of a central spiral, which presents the maximum diameter of the structure.

Unlike the previous zone, the objective of this central zone (5) is to stably contact the blood vessel wall. For a correct adaptation to the blood vessel (10), the central area (5) has a diameter greater than the diameter of the blood vessel (10). Thus, the diameter of the central area is preferably in the range 2-10 mm, depending on the blood vessel to be treated. In an even more preferred embodiment, the diameter may be in the range 5 - 8 mm. In this regard, for uses where the blood vessel (10) chosen is, for example, the internal carotid artery, the diameter of the central zone may be 7 mm.

Finally, following the central zone (5), the second peripheral zone (6), corresponding to the final part of the double conical spiral structure. Thus, the second peripheral zone (6) is formed by a second set of coils (7) and a terminal means (8).

The second set of spirals (7) present in the second peripheral zone (6) again forms a cone, where the base coincides with the central zone (5) of the structure, and the terminal means (8) is located downstream of the apex of the cone formed by the spirals. The terminal means (8) is configured for controlling the displacement, both during deployment and withdrawal, of the second set of coils. Preferably, the terminal means (8) is a means permanently attached to the second set of coils (7). By way of example, the half-finish (8) is a continuation of the same filament, preferably of nitinol, with a length of at least 90 cm.

Additionally, the arrangement of the double conical spiral structure achieves improved controlled removal of the embolic protection device (1) from the blood vessel (10). This reduces particle leakage during device removal, which is a substantial improvement over existing devices in the art.

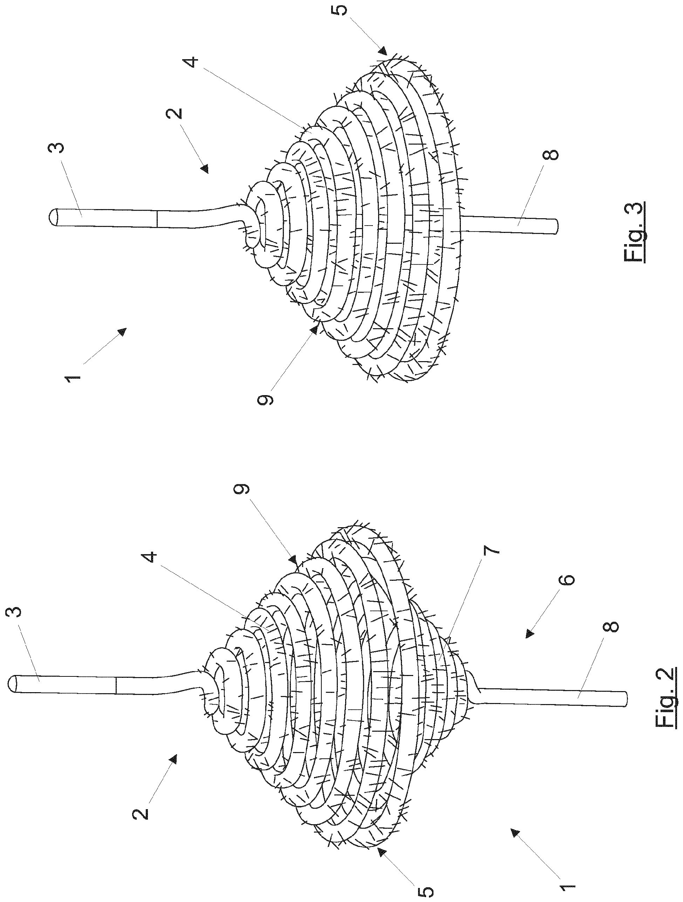

Below are Figure 2 and Figure 3, showing a perspective of a second preferred embodiment of the embolic protection device (1) in open and closed position, respectively.

In this second embodiment of the invention, the embolic protection device (1) presents a filament coated with fibers (9), i.e., it presents a multitude of fibers (9) on the outside of the filament along its entire length, with a preferred length of 100-200 microns. These fibers (9) are preferably anchored every 50 - 100 microns perpendicularly to the nitinol filament.

The presence of fibers (9) along the filament of the device (1) creates a tangle or network of fibers in which the blood particles are retained. In this way, it is possible to increase the capture capacity of the filament, which translates into an increase in the required distance between the turns of the first and second set of turns (2, 6). Thus, in a preferred embodiment, the spacing distance between the coils is 200 microns. In turn, the fibers (9) are made of nylon, although other equivalent materials, acceptable for the use of the device (1) in angioplasty procedures, could also be used.

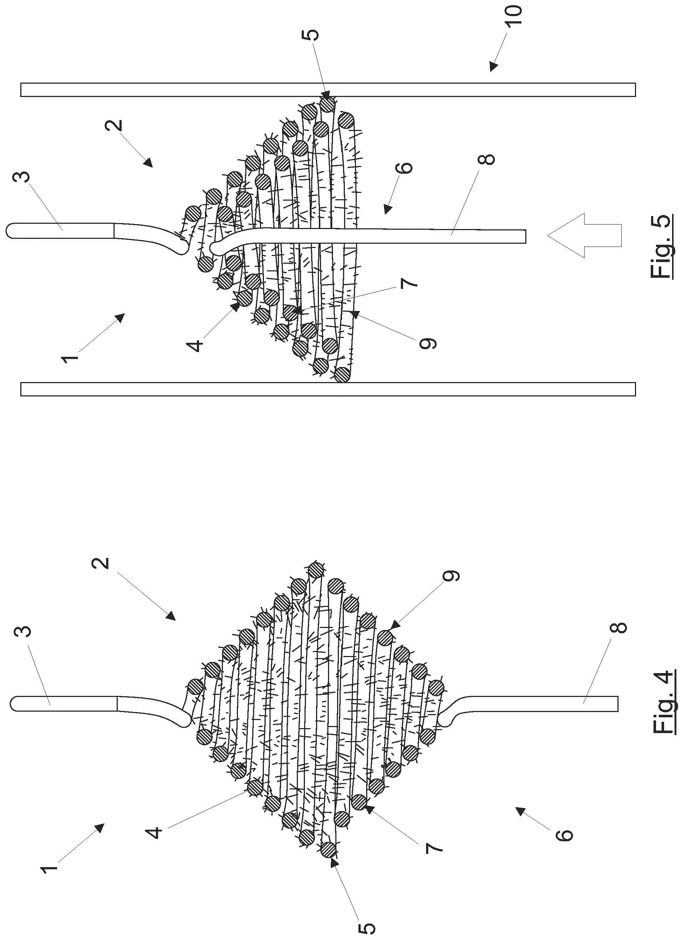

Finally, for a better detail of the displacement of the second set of coils (7), Figure 4 and Figure 5 show a section of the open and closed position of the embolic protection device (1), respectively. Specifically, the second embodiment of the invention is shown, being a device (1) featuring fibers (9) along the filament.

In addition to the above, the second set of coils (7) has the ability to move from an open position (Figure 4) to a closed position (Figure 5).

As shown in Figure 4, in the open position, the second set of coils (7) forms a cone of ascending diameter in the direction of blood flow, i.e. the base of this cone is coincident with the central zone (5) of the device and the apex of the cone, where the terminal means (8) is attached, is located in the position furthest away from it.

On the other hand, in the closed position, the second set of coils (7) reverses the orientation of the cone, forming in this case a cone with a descending diameter in the direction of blood flow. By reversing the orientation, the vertex of the cone, previously located the farthest away from the central zone (5), is introduced, this time, in this central zone (5).

The closed configuration of the embolic protection device (1), shown in Figure 5, features a double overlapping spiral that acts as a filter for solid particles in the bloodstream. This arrangement is achieved after coupling to the blood vessel (10) of central area (5), and allows to reduce the space between coils of the first and second set of coils (2, 6) to facilitate the capture of smaller particles detached during the angioplasty procedure.

The displacement, from the open position to the closed position of the second set of coils (7), can be generated by the action of the blood flow itself, which pushes the device (1), displacing the second set of coils (7). Alternatively, the terminal means (8) may be employed to facilitate moving the second peripheral zone from the open position to the closed position in a controlled manner.

In this Figure 5, additionally, the walls of the blood vessel are reflected (10). Thus, it can be seen how the central area (5) has a diameter greater than the blood vessel (10). In this way, an improvement in the adaptability of the device (1) is achieved by filtering the entire blood flow, and encompassing the full diameter of the blood vessel (10) in both deployment and withdrawal of the device (1)

Therefore, an embolic protection device (1) capable of highly efficient filtration for the capture and retention during device removal of small solid particles during angioplasty procedures is presented.

## Claims

1. An embolic protection device (1) during endovascular procedures inside a blood vessel (10)
**characterized in that** it comprises a filament preformed as a double conical spiral structure, which, in use, comprises
- A first peripheral zone (2) formed by a floppy (3) configured to align the device with the blood vessel (9) followed by a first set of coils (4) in the shape of a cone of descending diameter in the direction of blood flow,
- A central zone (5) formed by at least one central loop configured to contact the walls of the blood vessel, and
- A second peripheral zone (6), downstream of the central zone (5), formed by a second set of coils (7) and a terminal means (8) located at the end of the second set of coils (7) configured for controlling the displacement of the second set of coils,
wherein the second set of coils (7) is movable from an open position to a closed position, forming a cone of ascending diameter in the direction of blood flow inthe openposition , and a cone of descending diameter in the direction of blood flow through the central zone (5) in the closed position.

2. The embolic protection device (1) according to claim 1, wherein the filament has a thickness between 300-500 microns.

3. The embolic protection device (1) according to claims 1 to 2, wherein the floppy (3) has a length between 2 - 3 cm.

4. The embolic protection device (1) according to claims 1 to 3, wherein the central area (5) has a diameter of 2 - 10 mm.

5. The embolic protection device (1) according to claim 4, wherein the central area (5) has a diameter of 5 - 8 mm.

6. The embolic protection device (1) according to claim 5, wherein the central area (5) has a diameter of 7mm.

7. The embolic protection device (1) according to claims 1 to 6, wherein the terminal means (8) is a means permanently attached to the second set of coils (7).

8. The embolic protection device (1) according to claims 1 to 7, wherein the filament is made of nitinol.

9. The embolic protection device (1) according to claims 1 to 8, wherein the filament is a fibrillated filament.

10. The embolic protection device (1) according to claim 9, wherein the fibrillated filament has an anchor every 50 - 100 microns.

11. The embolic protection device (1) according to claims 9 to 10, wherein the fibers of the fibrillated filament are nylon.

12. The embolic protection device (1) according to claims 9 to 11, wherein the fibers of the fibrillated filament have a length of 100 - 200 microns.
